# EUROPEAN PATENT SPECIFICATION

(11) **EP 0 303 757 B1**
(45) Date of publication and mention of the grant of the patent: **01.06.1994**
(21) Application number: 88101435.1
(22) Date of filing: 02.02.1988
(51) Int. Cl.: A61B 5/028

(54) **Dual-thermistor thermodilution catheter**
Dual-Thermistor-Thermodilutionskatheter
Cathéter de thermodilution à thermisteur dual

(30) Priority: 17.08.1987 US 85552
(43) Date of publication of application: 22.02.1989
(73) Proprietor: B. Braun Melsungen AG, 34209 Melsungen (DE)
(72) Inventor: Griffin, Joseph C., Atco New Jersey 08004 (US); Skaggs, James L., Indian Mills New Jersey 08088 (US)
(74) Representative: Selting, Günther, Dipl.-Ing.

(56) References cited:
- EP-A- 0 182 363
- EP-A- 0 226 220
- WO-A-81/03614
- WO-A-84/00678
- US-A- 4 632 125

## Description

The present invention relates to catheters according to the preamble of claim 1 and particularly to balloon catheters that are used for thermo-dilution studies for providing diagnostic information from the temperature measurements to obtain the quantity of blood flow and the cardiac output.

Thermodilution catheters have been provided for the measurement of the temperature of mixed fluids in the heart and veins in order to provide important diagnostic information. Exemplary of the patent art relating to such catheters is the patent to Hassan H. Khalil U.S. Patent No. 4,217,910 and the patents and literature references referred to therein.

Thermodilution is an application of the calorimetric principle that, in a mixture of fluids at different temperatures, the heat lost by one fluid equals the heat gained by the other. For each fluid, the mathematical product of the temperature change, specific heat and mass is equal.

The recognized method for the study of blood circulation and a catheter device shown and described in EP-A-0226 220 involve producing a temperature change in the blood at one point (injectate port of the catheter) in the blood flow and measuring the temperature change at a second downstream point (thermistor probe on the inner tip of the catheter). Assuming that the measurement of the temperature change occurs at a point downstream of the heat source and that the blood's heat content is uniform, the measured change will reflect the amount of blood passing through the blood vessel.

In thermodilution studies heat is either removed from or added to the blood stream. One technique involves the injection of a cooler saline solution or bolus into the blood. In use, a known amount of a cold solution at a known temperature is injected into the right atrium or superior vena cava and the resultant temperature of the blood-bolus mixture is detected by a thermistor while the catheter is placed so that the thermistor bead is in the pulmonary artery. Cardiac output is inversely proportional to the integral of the observed temperature change. The accuracy of this method is dependent upon the accuracy of the measurement of the temperature of the injectate and the accuracy of the measured temperature of the resultant blood-injectate mixture. Assuming that the blood's heat content is uniform, the measured change in temperature provides a means of calculating the mass of the blood moving in a specific period of time and therefore the amount of blood flowing through the vessel which is a measure of the cardiac output of a particular patient.

The current methods and systems for conducting thermodilution measurements typically utilize external injectate bath temperature probes or external in line temperature sensors for measuring injection bolus temperature. These approaches immediately introduce an error in the calculations due to the fact that there will be some heat exchange and a change in temperature of the fluid in the bolus as it travels into and through the catheter when the catheter is placed in the body.

This condition created a need for an injectate temperature correction factor which could introduce error into the calculation of blood volume or cardiac output.

It is therefore an objective of the present invention to provide a multilumen thermodilution catheter which measures the temperature of the bolus as it is introduced into the blood stream so as to avoid inaccuracies that may occur in calculating cardiac output from the use of a bolus whose temperature is measured external of the body.

The invention solve's the mentioned problems of the prior art techniques by the multilumen catheter being provided with the features of claim 1.

A multilumen thermodilution catheter is described wherein a plurality of temperature sensing means are located adjacent the exterior in the catheter body at different distances laterally from the distal end of the catheter and each of said sensors is individually electrically connected to remote temperature measuring means by individual conducting means which communicate predominately through a single lumen in the catheter body and connecting assemblies, communicating with said lumen, to the remote temperature measuring means. In addition, a thermodilution catheter construction is provided which is unique and provides for a simplified method of construction wherein said catheter comprises an elongate multiple lumen catheter body having a distal end and a proximal end; manifold means located at the proximal end of said catheter body for separately connecting the lumens of said catheter body to sources of fluid, sources of gas and instrumentation and measuring means; a plurality of electrical connection means located in one of said lumens communicating with said instrumentation and measuring means and including at least two temperature sensor means; at least one of said sensor means being connected to at least one of said electrical connection means and being located adjacent the exterior surface of the catheter body near the distal end of said catheter body in a manner so as to be capable of sensing the temperature of fluids adjacent said exterior surface; at least one other of said temperature sensor means being connected to at least one other of said electrical connection means and located proximally of said first sensor means by means of passage of said one other second sensor means and attached electrical connection means from said one lumen containing said plurality of electrical connection means into a second of said lumens at a point adjacent said distally located sensor means and said second sensor means being located at a predetermined locus proximal of said distally located sensor means and affixed in said second lumen in a manner so as to be capable of sensing the temperature of fluid present in said second lumen.

### Brief Description of the Drawings

Fig. 1 is a partially broken plan view of a four lumen thermodilution catheter.

Fig. 2 is a cross-sectional view of the thermodilution catheter of Fig. 1 taken along the lines and arrows 2-2.

Fig. 3 is a partially broken plan view of a five lumen thermodilution catheter provided with an infusion or blood sampling lumen.

Fig. 4 is a cross-sectional view of the thermodilution catheter of Fig. 3 taken along the lines and arrows 4-4.

Fig. 5 is a partially broken plan view of another embodiment of a five lumen catheter.

Fig. 6 is a partially broken sectioned view of a portion showing the construction of the thermodilution catheter of the present invention.

Fig. 7 is a partially broken plan view of the some of the details of the construction shown in Fig. 6.

Fig. 8 is partially broken side view of the structure shown in Fig. 7.

Fig. 9 is a partially broken plan view of another of the details of the construction shown in Fig. 6.

Fig. 10 is a partially broken side view of the structure shown in Fig. 9.

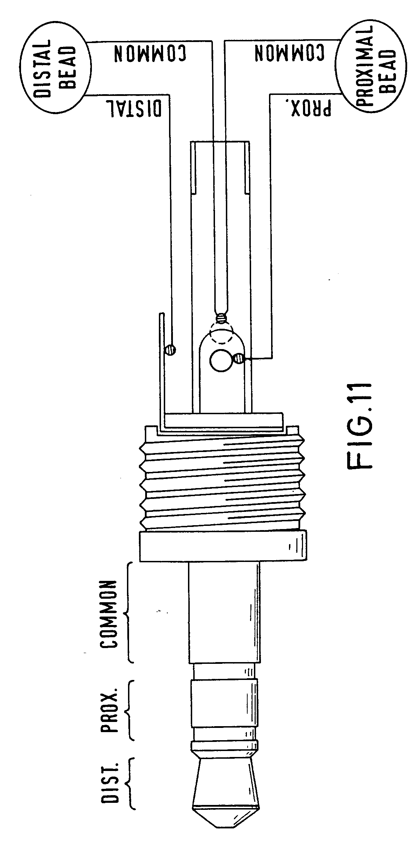

Fig. 11 is a plan view of the electrical connector wiring hook-up to distal and proximal thermistors.

### Detailed Description of the Invention

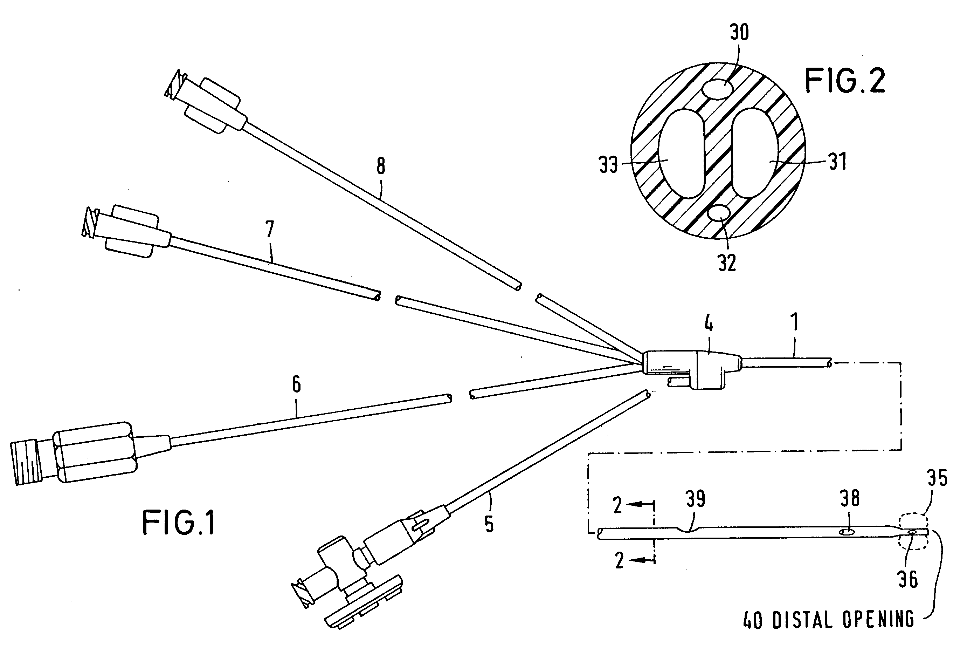

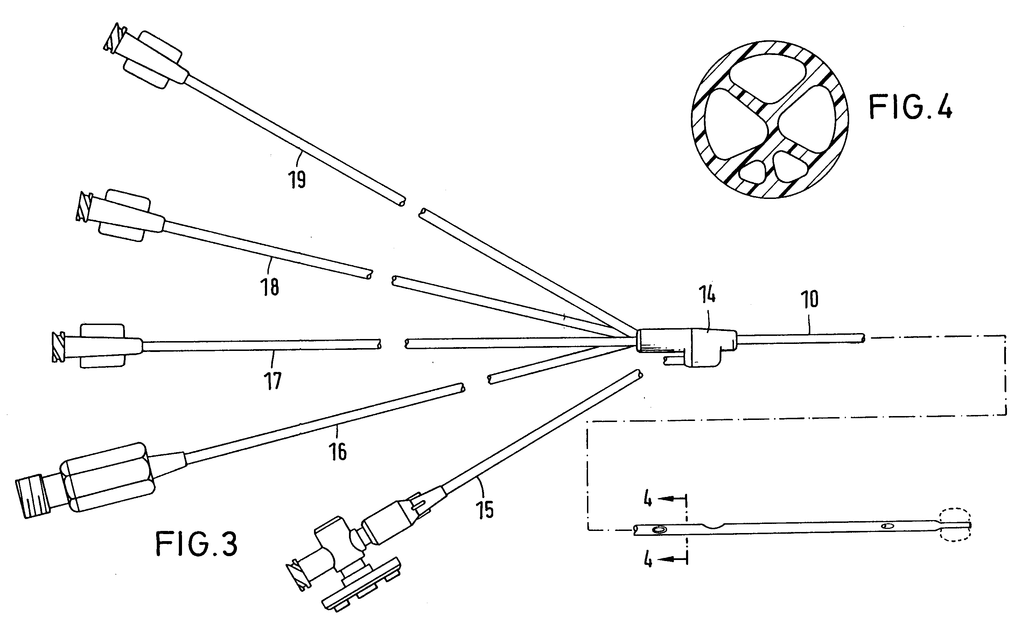

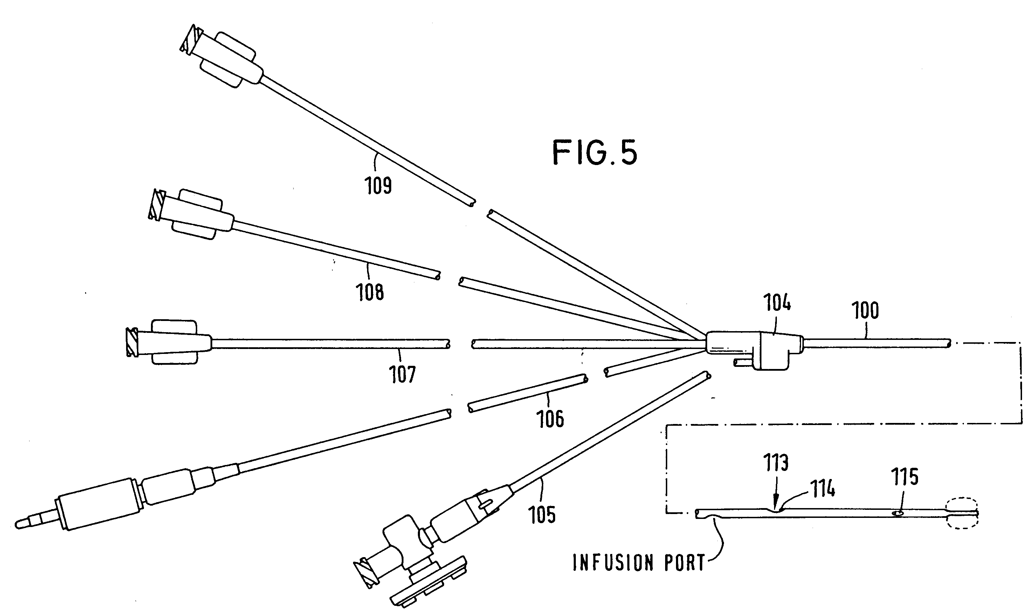

In Figs. 1, 3 and 5 multilumen catheter bodies 1, 10, and 100 respectively are provided on a balloon catheter. A manifold 4, 14, and 104 are each provided to provide communicating interconnection between the lumens of the catheter body with attachments including gas supply tube and assembly 5, 15, and 105 and thermistor electrical connector and wire containing tubes 6, 16 and 106. Additional lumen connecting assemblies 7 and 8 in Fig. 1; 17, 18 and 19 in Fig. 3 and 107, 108, and 109 in Fig. 5 can be provided for infusion of fluids such as a drug or thermodilution fluids or even blood sampling.

Typically the lumens shown in Fig. 2 provide the following functions. Lumen 32 communicates with gas supply assembly 5 through manifold 4 to inflate and deflate balloon 35 by a means hole 36 in catheter body 1 under the balloon location which communicates with the lumen 32.

Lumen 30 communicates with the electrical connector assembly 6 and is exposed at distal outlet 38 where a thermistor bead is cemented in a manner and location so as to be able to sense temperature at 38 without the lumen being exposed to blood or other fluids in the environment where the catheter is used. The lumen connecting assemblies 7 and 8 communicate with lumens 31 and 33 respectively which lumens independently communicate with the environment outside of catheter by means of openings 39 and 40 respectively. The most proximal of the openings 39 would typically be used for infusion of a bolus at a known fluid both temperature and the change in temperature would be sensed at 38 by the thermistor bead. From the values obtained cardiac output was calculated. The opening 40 to the remaining lumen could be used for the infusion of therapeutic or other fluid or drugs and measure intercardiac blood pressure or even used to withdraw blood samples if desired.

A variation on the foregoing, utilizing a five lumen catheter body, shown in section in Fig. 4 can be used where the additional lumen can be used for a variety of purposes, such as drug therapy, blood sampling and pressure monitoring.

The catheter shown in Fig. 5 provides the improvement of the present invention by maintaining the fluid infusion capability of the five lumen catheter of Fig. 3 while enabling greater accuracy in the measurement in temperature difference between the temperature of the bolus being introduced at opening 113, measured by thermistor bead 114 and the temperature of the blood bolus mixture at thermistor 115.

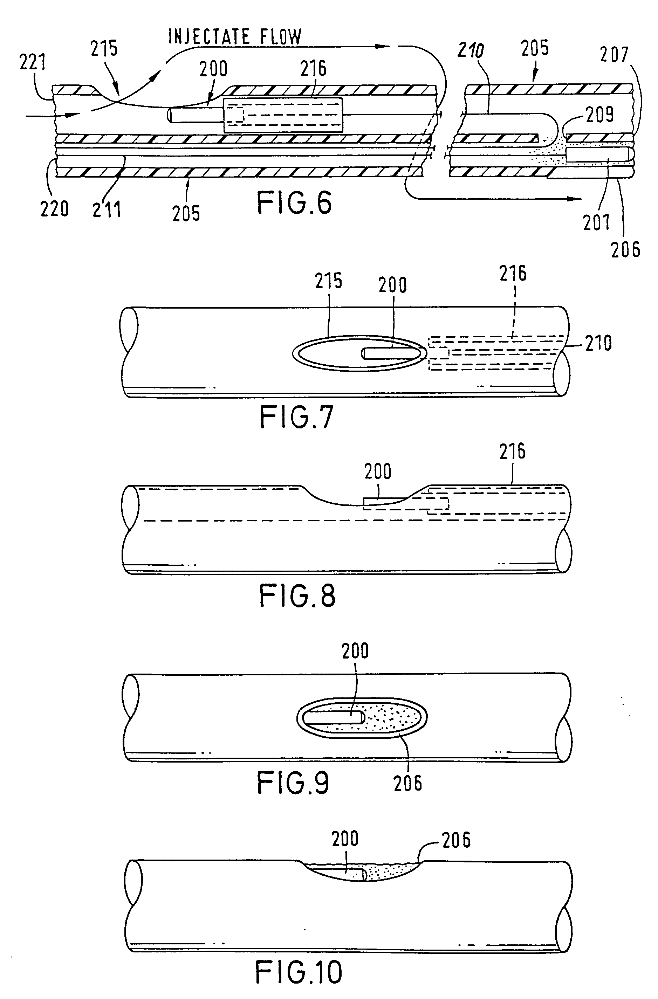

Referring to Figs. 6, 7, 8, 9, and 10, it will be more fully appreciated how the dual thermistor catheter of the present invention is constructed.

In Fig. 6, the thermistor beads are illustrated at 200 and 201. The connecting wires are respectively wire 210 and wire 211.

Both wires 210 and 211 are threaded down a single selected lumen designated 220 toward the proximal end of the catheter. The opening in the catheter wall 205 at 206 is the most distal opening. The lumen wall 207 separating lumen 220 from lumen 221 is provided with an opening 209 through which thermistor bead 200 and thermistor wire 210 are threaded back or distally until thermistor bead 200 is located adjacent the opening 215 which is provided in the exterior wall of lumen 221. During manufacture of the catheter the thermistor bead 200 and wire 210 is fitted with a sleeve 216 which is sized to be received in the lumen 221 in the position shown in Fig. 6. The sleeve 216 is adhesively affixed to the inside of the walls of the lumen 221 so as to provide a fluid tight fit. Placed as described, the lumen 221, proximal of the thermistor bead 200 still communicates with the outside of the catheter body through the opening 215 so that the lumen 221, with the appropriate connections to external fluid transport system can be used for the infusion of a bolus which will travel through the opening 215 and subsequently mix with the fluids, normally blood, external to the catheter. The thermistor bead 200 attached to appropriate instrumentation (not shown) will continuously measure the temperature of the bolus as it is introduced or as it exits the catheter lumen 221.

After placement of the thermistor bead 200, sleeve 216 and wire 210 the opening 209 in lumen wall 207 is sealed with an appropriate material such as urethane or other biocompatible material. The second thermistor bead 201 is then sealed into the position shown in Figs. 6, 9, and 10 with a bio compatible material.

In operation the bolus fluid and blood mixture with bolus fluid will flow generally in the manner depicted by the lines and arrows shown in Fig. 6. The temperature of the mixed fluids is then measured as before by the thermistor bead 201 and appropriate instrumentation. The temperature difference can then be used to accurately measure the cardiac output through the vessel being studied.

While the foregoing description if of a preferred embodiment of the present invention, it will be appreciated that other embodiments and variations are possible and within the scope of the appended claims which are to be interpreted in the light of the applicable prior art.

## Claims

1. A multilumen catheter for thermodilution measurement of cardiac output comprising:
an elongate multiple lumen catheter body (1) having a distal end and a proximal end;
manifold means (4) located at the proximal end of said catheter body (1) for separately connecting the lumens (30-33) of said catheter body (1) to sources of fluid, sources of gas and instrumentation and measuring means;
a plurality of electrical connection means located in one of said lumens (30) communicating with said instrumentation and measuring means and including temperature sensor means (201);
said sensor means (201) being connected to at least one of said electrical connection means (211) and being located adjacent the exterior surface of the catheter body (1) near the distal end of said catheter body (1) in a manner so as to be capable of sensing the temperature of fluids adjacent said exterior surface; characterized in that
at least one other of said temperature sensor means (200) being connected to at least one other of said electrical connection means (210) and located proximally of said first sensor means (201) by means of passage (209) of said one other second sensor means (200) and attached electrical connection means from said one lumen (220) containing said plurality of electrical connection means (210,211) into a second of said lumens (221) at a point adjacent said distally located sensor means (201) and said second sensor means (200) being located at a predetermined locus proximal of said distally located sensor means (201) and affixed in said second lumen (221) in a manner so as to be capable of sensing the temperature of fluid present in said second lumen (221).

2. The multiple lumen catheter of claim 1 wherein said temperature sensing means (201,200) includes a thermistor and electrical connection means (211,210) connected thereto for electrical attachment to temperature measuring instrumentation, said thermistor of said first sensor means (201) being affixed to said catheter at a point adjacent the exterior surface of the catheter body by means of a biocompatible affixing material.

3. The multiple lumen catheter of claim 2 wherein said multiple lumen catheter body contains an inflatable balloon (35) adjacent the distal end of said catheter body (1).

4. The multiple lumen balloon catheter of claim 3 wherein said at least one other second sensor means (200) is affixed proximal to said distal end of said catheter body in fluid tight relationship with the walls (205,207) of said second lumen (221) so as to prevent the passage of fluid in said second lumen (221) to any portion of said second lumen (221) distal to the point of affixation and said second lumen (221) communicates with the exterior of said catheter body at a point proximal to said point of affixation whereby fluids infused through said second lumen (221) distally of said catheter body will pass to the exterior of said catheter body at a locus adjacent to the thermistor of said second sensor means (200).

5. The multiple lumen balloon catheter of claim 4 wherein said multiple lumen catheter body (1) contains at least three lumens (30,31,32,33).

6. The multiple lumen balloon catheter of claim 4 wherein said multiple lumen catheter body (10) contains five lumens.

## Patentansprüche

1. Mehrlumen-Katheter zur Thermodilutionsmessung des Herzminutenvolumens, mit
einem länglichen Mehrlumenkatheter-Körper (1) mit einem distalen Ende und einem proximalen Ende;
einer am proximalen Ende des Katheter-Körpers (1) angeordneten Verteilervorrichtung (4) zur separaten Verbindung der Lumen (30-33) des Katheter-Körpers (1) mit Fluidquellen, Gasquellen und Instrumentier- und Meßvorrichtungen;
mehreren in einem (30) der Lumina angeordneten elektrischen Verbindungseinrichtungen, die mit den Instrument- und Meßvorrichtungen verbunden sind und eine Temperatursensoreinrichtung (201) enthalten;
wobei die Sensoreinrichtung (201) mit mindestens einer (211) der elektrischen Verbindungseinrichtungen verbunden und an der Außenfläche des Katheter-Körpers (1) nahe dem distalen Ende des Katheter-Körpers (1) derart angeordnet ist, daß sie die Temperatur von Fluiden nahe der Außenfläche aufnehmen kann,
**dadurch gekennzeichnet, daß**
mindestens eine weitere Temperatursensoreinrichtung (200) mit mindestens einer weiteren (210) der elektrischen Verbindungseinrichtungen verbunden und mittels des Durchtritts (209) der weiteren, zweiten Sensoreinrichtung (200) und angeschlossener elektrischer Verbindungseinrichtungen aus dem einen, die elektrischen Verbindungseinrichtungen (210,211) enthaltenden Lumen (220) in ein zweites Lumen (221) an einer Stelle in der Nähe der distal angeordneten Sensoreinrichtung (201) proximal zu der ersten Sensoreinrichtung (201) angeordnet ist, und daß die zweite Sensoreinrichtung (200) an einer vorbestimmten Stelle proximal zu der distalen Sensoreinrichtung (201) angeordnet und in dem zweiten Lumen (221) derart befestigt ist, daß sie die Temperatur von in dem zweiten Lumen (221) vorhandenem Fluid aufnehmen kann.

2. Mehrlumen-Katheter nach Anspruch 1, bei dem die Temperatursensoreinrichtung (201,200) einen Thermistor und mit diesem verbundene elektrische Verbindungseinrichtungen (211,210) zum elektrischen Anschluß an Temperaturmeßinstrumente aufweist, wobei der Thermistor der ersten Sensoreinrichtung (201) nahe einer an der Außenfläche des Katheter-Körpers befindlichen Stelle durch biokompatibles Befestigungsmaterial an dem Katheter befestigt ist.

3. Mehrlumen-Katheter nach Anspruch 2, bei dem der Mehrlumenkatheter-Körper einen aufblasbaren Ballon (35) an dem distalen Ende des Katheter-Körpers (1) aufweist.

4. Mehrlumen-Ballonkatheter nach Anspruch 3, bei dem die mindestens eine weitere, zweite Sensoreinrichtung (200) proximal zu dem distalen Ende des Katheter-Körpers in fluiddichter Beziehung zu den Wänden (205,207) des zweiten Lumens (221) befestigt ist, um den Durchtritt von in dem zweiten Lumen (221) befindlichem Fluid zu irgendeinem distal zu der Befestigungsstelle befindlichen Bereich des zweiten Lumens (221) zu verhindern, und das zweite Lumen (221) an einer proximal zu der Befestigungsstelle befindlichen Stelle mit der Außenseite des Katheter-Körpers in Verbindung steht, wobei distal zu dem Katheter-Körper durch das zweite Lumen (221) infundierte Fluide an einer dem Thermistor der zweiten Sensoreinrichtung (200) nahegelegenen Stelle zu der Außenseite des Katheter-Körpers gelangen.

5. Mehrlumen-Ballonkatheter nach Anspruch 4, bei dem der Mehrlumenkatheter-Körper (1) mindestens drei Lumen (30, 31,32,33) aufweist.

6. Mehrlumen-Ballonkatheter nach Anspruch 4, bei dem der Mehrlumenkatheter-Körper (10) fünf Lumen aufweist.

## Revendications

1. Un cathéter à plusieurs passages dans la mesure de thermodilution du débit cardiaque comprenant:
un corps de cathéter allongé (1) à plusieurs passages ayant une extrémité distale et une extrémité proximale;
un moyen à tubulure (4) disposé à l'extrémité proximale dudit corps de cathéter (1) pour relier séparément les passages (30-33) dudit corps de cathéter (1) aux sources de fluide, aux sources de gaz et à des moyens d'instrumentation et de mesure;
une pluralité de moyens de liaison électrique disposés dans un desdits passages (30) communiquant avec lesdits moyens d'instrumentation et de mesure et comprenant des capteurs de température (201);
lesdits capteurs (201) étant reliés à au moins un desdits moyens de liaison électrique (211) et étant disposés contigus à la surface externe du corps de cathéter (1) près de l'extrémité distale dudit corps de cathéter (1) de façon à pouvoir être capables de détecter la température des fluides près de ladite surface externe, caractérisé en ce que:
au moins un autre desdits capteurs de température (200) est relié à au moins un autre desdits moyens de liaison électrique (210) et disposé proximalement par rapport audit premier capteur (201) par le passage (209) dudit deuxième capteur (200) et des moyens de liaison électrique fixés à partir d'un passage (220) contenant ladite pluralité de moyens de liaison électrique (210, 211) dans un deuxième desdits passages (221) en un point contigu audit capteur (201) disposé distalement, ledit deuxième capteur (200) étant disposé en un lieu prédéterminé proximal dudit capteur (201) disposé distalement et fixé dans ledit deuxième passage (221) de façon à pouvoir être capable de détecter la température du fluide présent dans ledit deuxième passage (221).

2. Le cathéter à plusieurs passages selon la revendication 1, dans lequel lesdits capteurs de température (201, 200) comportent un thermistor et des moyens de liaison électrique (211, 210) qui y sont reliés pour la fixation électrique au moyen d'instrumentation de mesure de température, ledit thermistor dudit premier capteur (201) étant fixé audit cathéter en un point contigu à la surface externe du corps de cathéter au moyen d'un matériau de fixation biocompatible.

3. Le cathéter à plusieurs passages selon la revendication 2, dans lequel ledit corps de cathéter à plusieurs passages renferme un ballon gonflable (35) de l'extrémité distale dudit corps de cathéter (1).

4. Le cathéter à ballon à plusieurs passages selon la revendication 3, dans lequel au moins ledit autre deuxième capteur (200) est fixé proximalement par rapport à ladite extrémité distale dudit corps de cathéter en relation fluidique étroite avec les parois (205, 207) dudit deuxième passage (221) de façon à empêcher le passage du fluide dans ledit deuxième passage (221) à toute partie dudit deuxième passage (221) distale par rapport au point de fixation et ledit deuxième passage (221) communique avec l'extérieur dudit corps de cathéter en un point proximal par rapport audit point de fixation si bien que les fluides infusés au travers dudit deuxième passage (221) distalement par rapport audit corps de cathéter passent à l'extérieur dudit corps de cathéter en un lieu contigu au thermistor dudit deuxième capteur (200).

5. Le cathéter à ballon à plusieurs passages selon la revendication 4, dans lequel ledit corps de cathéter (1) à plusieurs passages renferme au moins trois passages (30, 31, 32, 33).

6. Le cathéter à ballon à plusieurs passages selon la revendication 4, dans lequel ledit corps de cathéter (10) à plusieurs passages renferme cinq passages.
